# EUROPEAN PATENT SPECIFICATION

(11) **EP 1 523 357 B1**
(45) Date of publication and mention of the grant of the patent: **19.03.2008**
(21) Application number: 03763562.0
(22) Date of filing: 08.07.2003
(51) Int. Cl.: A61M 5/30

(54) **NEEDLE-FREE INJECTION DEVICE**
NADELLOSE INJEKTIONSVORRICHTUNG
DISPOSITIF D'INJECTION SANS AIGUILLE

(30) Priority: 11.07.2002 WO PCT/IB02/02703; 28.10.2002 WO PCT/IB02/04494
(43) Date of publication of application: 20.04.2005
(73) Proprietor: TecPharma Licensing AG, 3401 Burgdorf (CH)
(72) Inventor: NERACHER, Arnold, CH-1247 Anières (CH)
(86) International application number: PCT/CH2003/000454
(87) International publication number: WO 2004/006998

(56) References cited:
- WO-A-02/32484
- DE-B- 1 231 966
- FR-A- 2 188 736
- FR-A- 2 324 494
- GB-A- 1 048 484
- GB-A- 1 277 055
- US-A- 6 053 890

## Description

### Background of the Invention

The present invention relates to a needle-free injection device for transdermal injection of a liquid. US-6,053,890 discloses a device according to the preamble of claim 1.

In international patent application WO 01/47586 A1, a propulsion system for a needle-free injection device, comprising a primary source of stored potential energy in the form of a compressed liquid, is described. Preferred compressible substances for the primary energy source are chosen from the family of polysiloxanes in view of their high compressibility compared to other liquids or solids. The provision of a compressed liquid or solid as a primary source of potential energy for propelling at liquid to be injected is advantageous over systems using compressed gas or mechanical springs because of the significantly higher energy density that the compressed liquid or solid offers. Springs, for example, requires large dimensions to obtain the required propulsion energy to ensure that a patient's skin is pierced, and a relatively large liquid jet diameter in order to ensure sufficient power of the jet. Systems using compressed gas are limited by the maximum pressure of the gas until a change of state to the liquid form, which defines the maximum pressure of the propulsion system. The risk of explosion of compressed gas systems is also a safety concern.

WO 02/32484 A1 discloses a needle-free injection device using an elastomeric spring as energy source.

The propulsion system described in the aforementioned publication further comprises a secondary source of potential energy generating a lower pressure than the primary source. This enables the injection depth to be accurately controlled, in particular to limit the depth of liquid delivery once the skin has been pierced by the initial high pressure jet. This is important in applications requiring intradermal or subcutaneous delivery. Examples of the secondary source of potential energy include a metal spring, a gaseous substance, or a compressible liquid substance similar to the substance used in the primary energy source.

The use of a liquid polysiloxane compressed in a container as an energy source enables the propulsion system to be very compact, safe and relatively inexpensive. Nevertheless, the high pressure of the liquid polysiloxane in the container requires effective seals and a pressure resistant container that impinge on the manufacturing costs. In this respect, there is a need to provide a needle-free injection device that is reliable and compact, yet inexpensive to manufacture.

### Summary of the Invention

Considering the above, an object of the invention is to provide a needle-free transdermal injection that is safe, reliable, inexpensive to produce and able to inject liquid at a controlled depth below the skin.

It is advantageous to provide a needle-free injection device that injects painlessly and with minimal tissue damage.

It is advantageous to provide a needle-free injection device that is light and compact.

It is advantageous to provide a needle-free injection system that is versatile and may be embodied in single-use disposable or multi-use injection devices.

Objects of the invention have been achieved by providing the injection device according to claim 1.

Disclosed herein is a needle-free transdermal injection device comprising a source of potential energy and a mobile force transmitting member (10, 10') for applying sufficient pressure on a liquid to be injected (2) for propulsing the liquid to be injected (2) through a nozzle orifice (24,24') at a velocity sufficient for transdermal injection, wherein the source of potential energy primarily comprises a solid elastic material or plurality of materials exhibiting a non-linear characteristic of compression force F as a function of compression displacement x, whereby the ratio of the compression force F as a function of the compression displacement x increases with the compression displacement x. Such materials include reticulated polymers, such as polyurethane, which are light and cost effective. The material or material form or are part of a spring member essentially in the form of a block elastically compressed between a wall portion of the mobile force transmitting member and an opposed wall portion of a housing or supporting structure of the injector. Advantageously, not only is the energy density of the compressed material of the spring member block greater than the energy density of conventional coil or other metal springs, or compressed gas propulsion systems; but the non linear force characteristic provides a high initial peak pressure to propel the liquid through a very fine nozzle orifice with sufficient velocity to pierce skin, followed by a rapid drop to a lower pressure level that ensures delivery of the liquid at a controlled depth below the skin. Moreover, the injector according to the invention does not have any sealing problems as in gas or liquid based propulsion systems, and there is no risk of explosion as in gas based systems.

It is also easy to adjust to the optimum injection characteristics by varying various parameters of the propulsion system spring member, such as the diameter, compressed distance and composition of materials thereof, without changing the overall design of the injector. The propulsion system of the invention is also extremely versatile and can easily be adapted for use in single use disposable injectors, which may be provided with low cost plastic housings, or for use in re-usable (multi-use) injectors.

Advantageously, a particularly versatile, compact, low cost and safe needle free injection device, that can accurately deliver a liquid transdermally to a required depth, is provided.

Further objects and advantageous aspects of the invention will be apparent from the following description, claims and accompanying drawings.

### Brief Description of the Drawings

Fig. 1 is a longitudinal section in perspective of an injection device comprising a propulsion system according to this invention, in a loaded position;
Fig. 1a is a londitudinal section of a front portion of the injection device of figure 1;
Fig. 1b is a londitudinal section of a central portion of the injection device of figure 1;
Fig. 1c is a londitudinal section of a rear portion of the injection device of figure 1;
Fig. 1d is a partial section of a front portion of the injection device of figure 1;
Fig. 2 is a longitudinal section of the injection device with the propulsion system in an actuated or unloaded position;
Figures 3a to 3d are perspective views of various spring elements usable in a propulsion system of an injection device according to the invention;
Fig. 4a is a graph illustrating the relationship of propulsion force of the propulsion system of the injection device as a function of the axial displacement of the propulsion system piston, the different curves related to different elastic materials used in the propulsion system;
Fig. 4b is a graph illustrating the relationship of pressure of the liquid to be injected as a function of the volume of liquid injected for an injection device according to the invention; and
Fig. 5 is a longitudinal section of a single-use disposable injection device, prior to actuation, which is not within the scope of the present invention.

### Detailed Description of the Invention

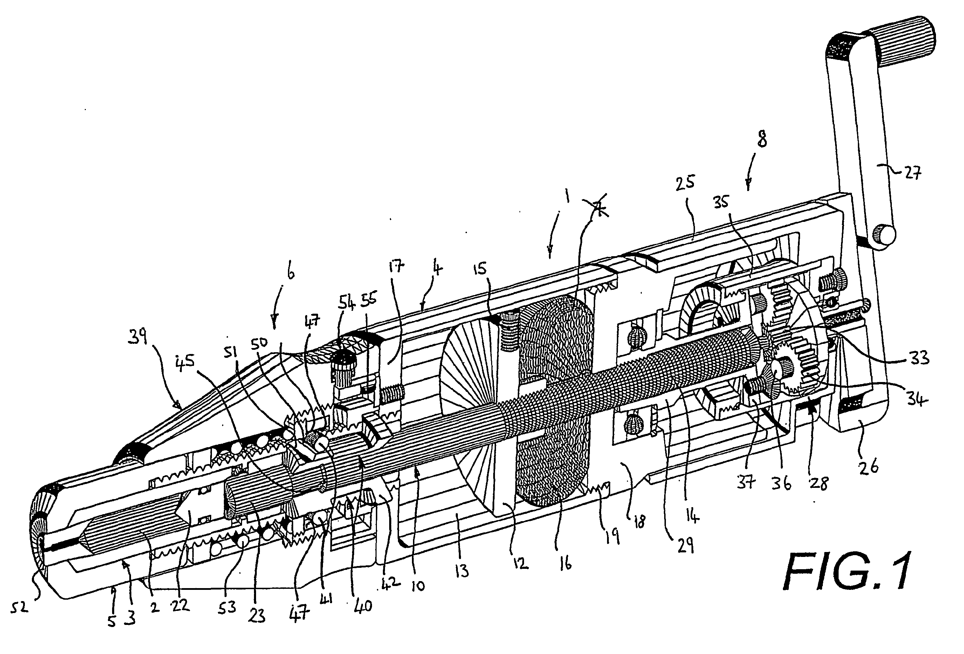

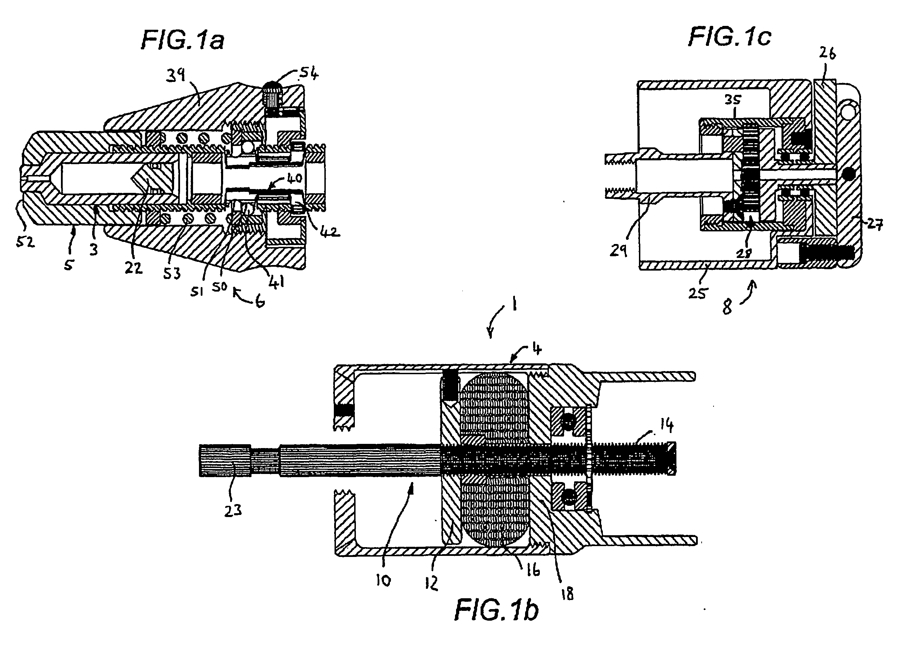

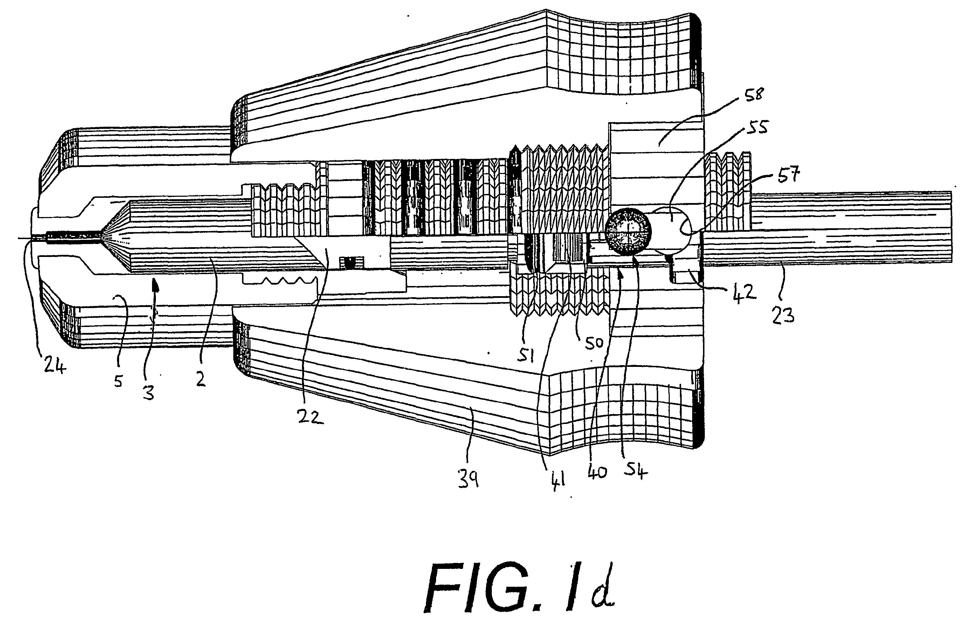

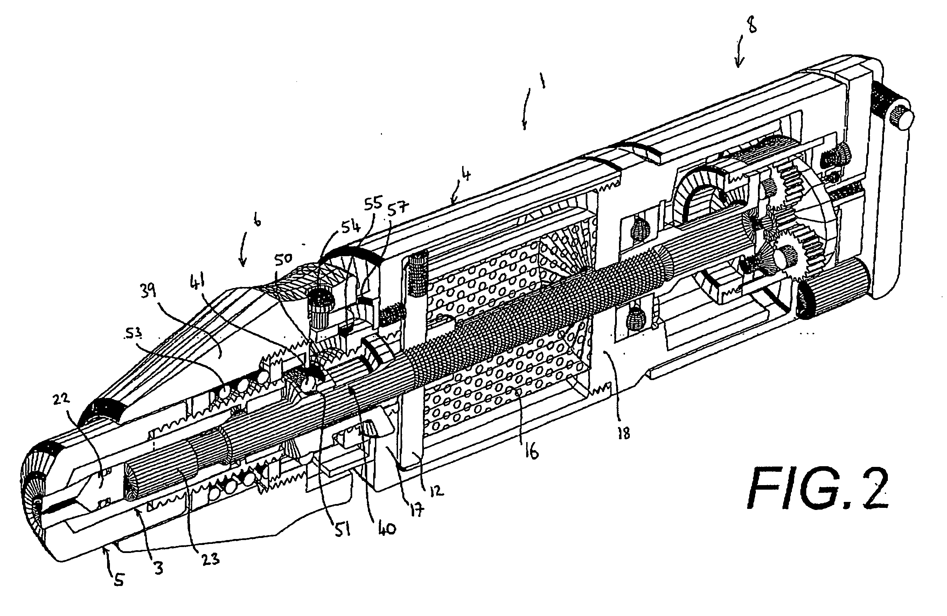

Referring to Figures 1 and 2, a re-usable needle-free injection device is shown comprising a propulsion system 1, an actuator system 6, and means in the form of a removable cap 5 for mounting a disposable capsule 3 thereto for the transdermal administration of a liquid 2 contained in the capsule.

The propulsion system comprises a housing 4, a source of potential energy 7, a force generating mechanism 8, and a mobile force transmitting member 10. The mobile force transmitting member comprises a propulsion system piston portion 23, a mobile force wall portion 12, and a force generating engagement portion 14 in the form of a screw thread portion coupled to the force generating mechanism 8. The potential energy source comprises a solid elastic spring member 16 mounted between the mobile force wall portion 12 and a housing wall portion 18. To generate the potential energy used for injection, the spring member is elastically compressed between the force wall portions 12, 18 by axially displacing the mobile force transmitting member 10 with the force generating mechanism 8 as shown in figures 1, 1c and 2.

In this example, the piston portion 23 and engagement portion 14 form substantially a rod extending along the central axis of the injection device, the mobile force wall portion being attached to the rod, or integrally formed therewith. It is however possible to provide other constructions driving the mobile force wall portion toward the housing wall portion. The mobile force wall portion is in this embodiment in the form of a plate positioned in a cavity 13 of the housing 4. The mobile force wall portion 12 and housing 4 may be provided with complementary polarising or keying means to prevent rotation of the plate 12' about its central axis with respect to the housing, due to the torque exerted on the rod by the pressure generating mechanism. The keying means comprises for example a projection 15 of the wall portion 12 received in a complementary axial groove in the housing.

In the unloaded position, as shown in figure 2, the mobile force transmitting member 10 is stopped in a forwardmost position with respect to the housing, whereby the spring member is not entirely relaxed. In other words, the spring member is compressed with a certain force between the wall portions in the unloaded position, so that the piston portion exerts sufficient pressure on the liquid being injected out of the capsule or cartridge 3 at the end of injection to ensure that all the liquid is delivered transdermally at the required depth. It is possible to provide an injector with means to adjust the travel of the mobile pressure wall portion with respect to the housing to change the end of injection spring force. One could for example provide an axially adjustable stop (not shown) extending between the front housing wall portion 17 and the mobile pressure wall portion 12, or adjust the position of the front housing wall portion 17 with respect to the rear housing wall portion 18 - in this example by screw threading one housing portion with respect to the other via the screw coupling 19.

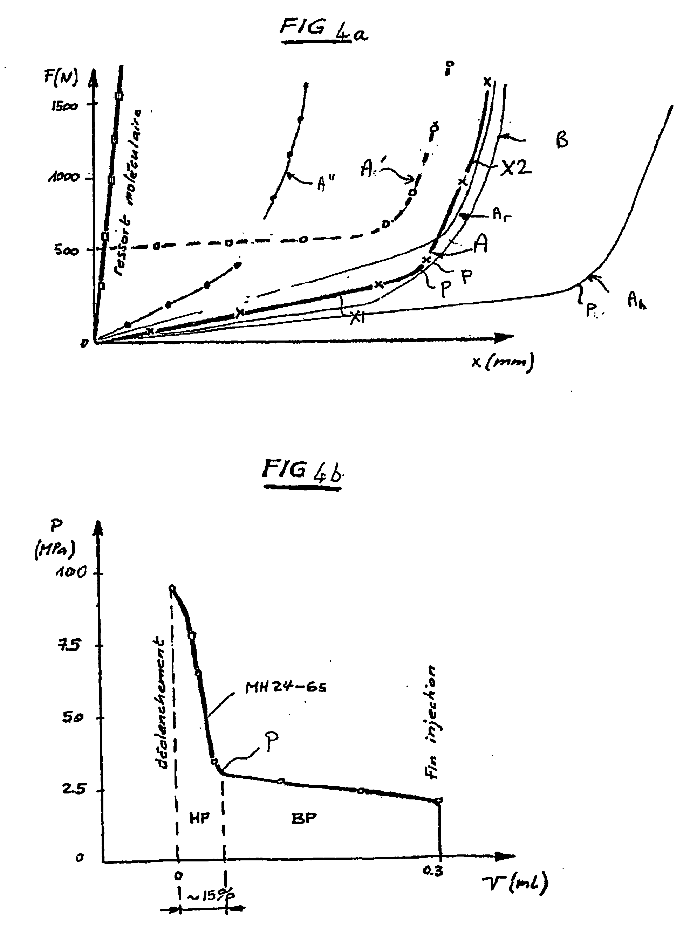

In the present invention, the spring member is essentially in the form of a block of elastically compressed material or materials with a marked non-linear force characteristic of compression force F(x) versus displacement x in the direction of deplacement, whereby the slope of the function F(x)/x increases with the displacement x as best seen in figure 4a, curves A, B and C. In other words the elastic module of the spring element increases with compression of the spring member as the wall portions move together. This non-linear spring characteristic is very advantageous since the propulsion system provides a high initial or primary force to produce a high speed micro-jet able to pierce a patient's skin, followed by a lower secondary force over a longer displacement to ensure that the micro-jet does not penetrate the patient's tissue too deep once the skin has been pierced.

Curve A of figure 4a illustrates the force F(x) required to advance the mobile wall portion 12 as a function of its displacement (x) from the unloaded position (0), the first portion X1 of the curve A having a gradient F(x)/x less than the second portion X2. Point P indicates roughly the transition point or zone at which the first and second portions of the curve meet. The steep gradient of the second portion X2 provides a source of energy for initial high pressure injection, for example peaking at 600 to 1000 bars as shown in figure 4b, followed by liberation of energy at relatively low pressure, for example around 200 bars, when the position of the mobile wall portion is in the region of the first portion X1 of the curve F(x). This double injection pressure stage is very advantageous since it enables the injection depth to be accurately controlled, for example to deliver liquids such as insulin or other medicines intradermally or subcutaneously. The initial high pressure enables a very fine high speed (for example supersonic) liquid jet to be formed to pierce skin, followed by the lower pressure second stage jet to deliver the liquid at a controlled depth below the outer surface of the skin, avoiding excessive penetration that would ensue if the initial high pressure were maintained over a longer period.

The position of the transition point P between first and second curve portions, the gradients of the portions X1 and X2 , and the force at x=0 can be adjusted by the selection of the material or combination of materials and the geometry and dimensions of the spring member.

Preferred materials for the spring member according to this invention, that exhibit a non-linear multi-stage compression force characteristic as discussed above, and that can store a high energy density in the elastic domain, are reticulated polymers, such as polyurethanes and various rubbers such as vulcanised natural rubber, isobutylene rubber, nitrile rubber, propylene oxide rubber, silicone rubber (SILASTIC), fluorsilicone rubber, polynoborene rubber (NORSOREX), EPDM rubber, KEL-F. In reticulated polymers, the long molecular chains are held together by molecular bonds in a network that is in a low state of entropy when relaxed, the entropy increasing as the material is crushed. Upon relaxing a block of reticulated polymer compressed between two parallel plates, as long as the force remains in the elastic domain of deformation for the particular material, the block recovers its original shape. In the present invention, the spring member does not act as a molecular spring compressed in a container as is the case in the system described in international patent application WO 01/47586 A1 where polysiloxane is compressed in a container. Rather, in the present invention, the spring member is free to expand laterally, and lateral space is provided therefor in the injection device housing 4, as best seen in figures 1 and 2. The diameter of the cavity of the housing is sufficient to allow a diameter increase of the spring block of about 20 to 30% as it is compressed. The central rod 14 extending through the spring member block 16 has the important function of acting as a guide to ensure uniaxial compressing and after a biaxial compressing of the spring member and to prevent it from deforming in a non-axisymmetrical manner.

The spring member material may advantageously have a cellular structure as in a foam material whereby the material is filled with small air pockets or cells. An example of cellular material with advantageous properties is cellular polyurethane such as the trademark product CELLASTO type MH-24-65. Curves A and A' of figure 4a correspond to the crushing of a cylindrical block of CELLASTO (dimensions: 40mm height and 25mm diameter in the normal state). In an initial compression phase X1 when the cells collapse, the spring force F as a function of displacement x is essentially linear and has a low gradient. Once the cells of the material are completely flattened, the spring force increases more rapidly and the gradient of the function F=f(x) increases. Cellular reticulated polymers advantageously have a greater elastic compression displacement range than non-cellular reticulated polymers, thus extending the low pressure portion X1 of the force function F=f(x), which is useful for the injection of fairly large volumes of liquid, for example in the range of 0.2 to 0.8ml. In the present invention, it is possible to have an additional energy source in the form of a conventional spring for example, to generate additional energy for the low pressure injection phase. Unless particularly large volumes of liquid are to be injected, an additional energy source will for most cases not be useful, particularly considering the additional cost, weight and size. An important advantage of the energy source according to this invention is that a low cost and compact spring block is able to generate a high peak pressure in an initial injection phase, followed by a lower pressure in a subsequent injection phase, the pressures being largely sufficient to propel liquid through a very fine nozzle orifice, for example having a diameter in the range of 50 to 100 microns The small nozzle orifices that may be employed in injection devices according to this invention reduces tissue damage and pain compared to conventional needle free devices employing nozzle orifices having diameters around 180 to 280 microns in view of the lower pressures generated by conventional propulsion systems based on compressed gas or metal coil springs.

Figure 4b illustrates the pressure, in the cartridge 3 of the liquid 2 to be injected in an injection device as shown in figures 1 to 3 with a cylindrical spring member of cellular reticulated polyurethane, during injection. In this example, the parameters are:
diameter of the spring member = 20mm
spring axial length = 80mm
nozzle orifice diameter = 80 microns
volume to be injected = 0.5ml

As shown in figure 4b, the initial peak pressure in the liquid is about 100 Mpa, dropping to 30 Mpa after about 15% of the volume of liquid has been injected, the low pressure injection ending at about 20 Mpa.

A cellular reticulated polyurethane such as the product CELLASTO is compressed at around 100N/cm² when the cells are completely flattened, and can be compressed elastically up to a maximum of around 300 to 500N/cm². By comparison, a metal coil spring of the same diameter (e.g. 20mm) and height (e.g. 50mm), generates the equivalent of around 100N/cm² when fully compressed, and does not exhibit a multi-stage pressure drop effect.

The spring member of the present invention made of reticulated polymer is particularly cost effective to produce since it can be moulded into the desired shape and in view of the low cost of polymers in general. Reticulated polymers such as reticulated polyurethanes are also advantageous in view of their low weight density for the amount of compression energy that can be stored (in other words there is a high ratio of stored compression energy per unit weight). Moreover, such polymers are operational in the range of temperatures from -20 to 80°C without significant variation in their physical properties, in particular elastic properties.

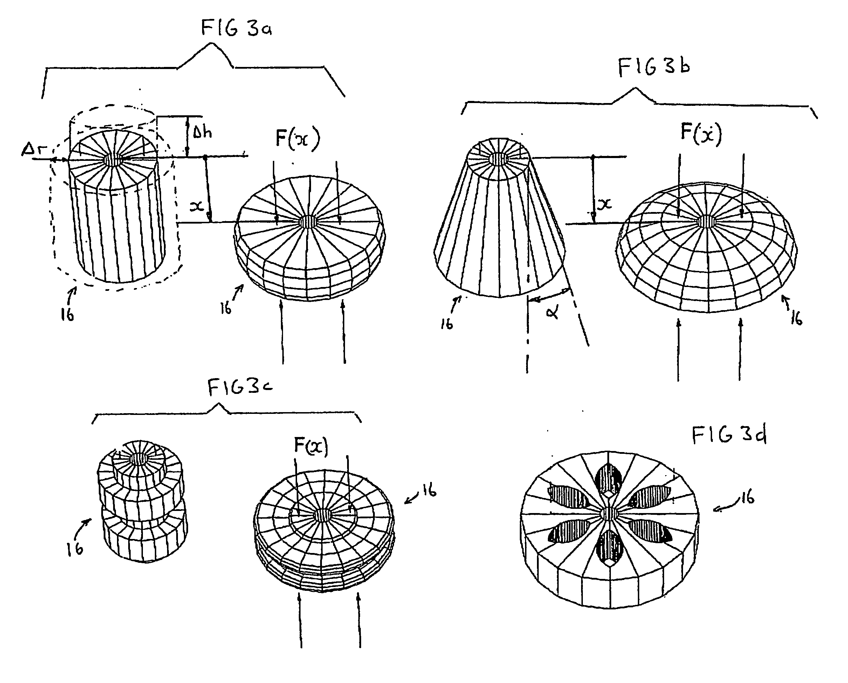

The block may be made of a single material, as a single piece, as represented in figures 1 to 3, or as a stack or combination of pieces of a same or of different materials. The combination of different materials or pieces with different geometries as shown in figures 3a to 3d enables the characteristic of force versus displacement of the propulsion system to be adjusted within a wide range of possible curves to optimise the speed of the liquid micro-jet taking into account the volume to be injected and the depth of transdermal delivery. The propulsion force characteristic as a function of displacement F=f(x) can for example be adjusted by varying the following design parameters:
- diameter of the spring member: effects the magnitude of the force by changing the slope of the function F=f(x) - an increase in radius □r as shown in figure 3a increases the slope of F=f(x) as illustrated by curve Ar in the graph of figure 4a
- axial height of the spring member : affects the travel of the spring member and displaces the point of transformation P between primary to secondary portions of the curve - an increase in axial height □h shifts the point P to the right as illustrated by the curve Ah of figure 4a
- varying diameter of the spring member as a function of height: effects the slope of the function F=f(x): a conical increase in diameter as shown in figure 3b reduces the curvature of the function around the point of transformation P as illustrated by the curve B of figure 4a
- prestressing the spring member: raises and flattens the first portion X1 of the curve as shown by the curve A' in figure 4a
- various other parameters also affect the characteristic of the function F(x) of the propulsion system, such as the type of material or combination of materials forming the spring member - the curve A" in figure 4a for example represents the function F=f(x) for a cylindrical spring member block as shown in figure 3a, made of natural vulcanised rubber
- the spring block member may also be made of a complex shapes with internal cavities as shown in figure 3d or of a stack of discs of different reticulated polymers and/or of different diameters as shown in figure 3c

Thus, the propulsion system according to the invention may be advantageously varied within a large range of values to optimise injection taking account of the amount of liquid to be injected, the desired depth of transdermal delivery, the desired injection time, and the skin resistance level (which depends for example on the age of the person, the location of injection on the body, and skin type).

The front end of the injector housing is provided with a removable cap 5 engaging a threaded portion 21 for releasably mounting the capsule 3 containing the liquid to be injected in the injector housing. Other releasable fixing means could however be provided, such as a bayonet type connection or releasable spring latches. A rear end of the capsule is sealingly closed by a sealing piston 22 that is driven by the propulsion system piston 23 on actuation of the device thereby propulsing the liquid 2 through the capsule nozzle orifice 24. The capsule sealing piston 22 may be provided at its front end with a cone shaped elastic portion in order to ensure that substantially all the liquid to be injected is propelled out of the capsule.

The force generating mechanism 8 is mounted to the rear end of propulsion system and comprises a first grip portion 25, a second grip portion 26 with a foldable lever portion 27 and a planetary gear mechanism 28 driving a threaded drive tube 29 engaging a complementary threaded portion 14 of the mobile pressure transmitting member 10. As the first grip portion 25 is turned, the threaded drive tube 29 turns and drives the mobile pressure wall portion 12 axially rearwards, thus compressing the spring member block 16 against the rear housing wall portion18.

The second grip portion 26 and foldable lever 27 is fixed to a central pinion 33 that engages satellite gear wheels 34 that in turn engage an outer annular crown gear 35 integral with or fixed to the first grip portion 25. The axes 26 of the satellite gears 34 are mounted pivotly on a flange 37 rigidly fixed to the threaded drive tube 29. In the example illustrated in the figures, the step down gearing ratio is approximately 4:1, i.e. an operator must turn the lever portion approximately 4 full turns for the threaded drive tube 29 to effect one full turn. In use, a human operator may initially turn the first grip portion 25 to load the propulsion system partially until the torque is too high for the operator, whereby the lever portion 27 may then be folded out, as shown in figure 1, and turned to complete loading of the propulsion system. In this manner, the propulsion system can be quickly loaded by a human operator, while enabling a high final compression level to be attained in the propulsion system.

The pressure generating means may also be driven by an electric motor (not shown) coupled to the central pinion 33. The motor may either be permanently mounted or integrated into the rear end of the injection device, or be provided as a separate unit that removably engages the central pinion 33, for example much like an electric screw driver.

The actuation system 6 comprises an actuator member 39, one or more piston retaining elements 40, and one or more blocking inserts 41 mounted between the actuator member and the piston retaining elements.

The retaining elements 40 are pivotly mounted and axially retained in the injector housing 4 by a wall portion 42 thereof extending radially outwards at a rear end of the retaining element and inserted with some play in a corresponding cavity 43 in the housing. The retaining elements however could also be attached to the housing with an elastic hinge to allow the retaining elements to pivot in order to release the propulsion system piston 23. In this example, the two opposed retaining elements are in the form of angular segments, for example roughly 90° segments, of a tubular part. Thus, two pairs of segments can be produced by two perpendicular cuts through the axis of the initial tubular part. The retaining element comprises an inwardly projecting retaining shoulder 44 at a front end thereof engageable with a complementary shoulder 45 of the propulsion system piston 23, in order to retain the propulsion system piston in the loaded position prior to actuation.

The blocking insert 41 is postioned between an outer camming surface 46 of the retaining element and an inner camming surface 47 of the actuator member 39. The inner camming surface 47 is bounded at front and rear ends thereof by front and rear ledges 48, 49 respectively that limit the relative axial displacement of the blocking insert 41. The camming surface of the retaining element 40 has a high point locking surface portion 50 and a low point release surface portion 51. In the loaded position, as shown in figures 1 and 1a, the blocking insert 41 is positioned between the high point locking surface portion 50 and the inner camming surface 47 of the actuator member 39 thereby blocking outward pivoting of the retaining element 40 such that the retaining shoulder thereof remains in engagement with the complementary shoulder 45 of the propulsion system piston 23. The injection device is actuated by displacing the actuator member 39 axially toward the front or injection end 50 of the device, whereby the blocking insert 41 rolls along the retaining element camming surface to the low point release surface portion 47 as shown in figure 2a, such that the retaining elements pivot radially outwards and release the propulsion system piston 23. It may be noted that the retaining elements will tend to pivot outwards about their pivot attachment ends 42 in view of the moment created by the force exerted by the propulsion system piston 23 on the retaining shoulders 44 of the retaining elements 40.

The blocking insert 37 is advantageously an element that rolls along the camming surface in order to reduce frictional force, particularly in view of the high axial force exerted by the propulsion system. The blocking element may be in the form of a solid cylinder, but could also be in the form of a plurality of balls or of a cylinder formed of a spring wire coil.

The pressure retaining and release means may further comprise a return spring 53 that axially biases the actuator member and, as a consequence the blocking insert, into the retaining position shown in figure 1, when the propulsion system piston is drawn back after actuation for reloading. A safety lock in the form of a button 54 for example, may be provided to axially lock the actuator member 39 and prevent indavertent axial displacement of the actuator member. The safety lock may also serve to indicate to a human operator when the injection device is in the loaded position or has been actuated. In this example, the safety lock comprises a locking key portion 55 biased by means of a spring 56 into a complementary key cavity in a cylindrical wall portion 58 of the injection device housing. In the retaining or loaded position as shown in figure 1, the locking key portion 55 engages in the complementary key cavity 57 and axially locks the actuator member 39 and the button 54 protrudes slightly beyond the outer surface of the actuator member 39. In the unlocked and actuated positions, the button 54 is in a depressed position below the outer surface as seen in figure 2, and the key portion 55 is disengaged from the complementary locking cavity 57.

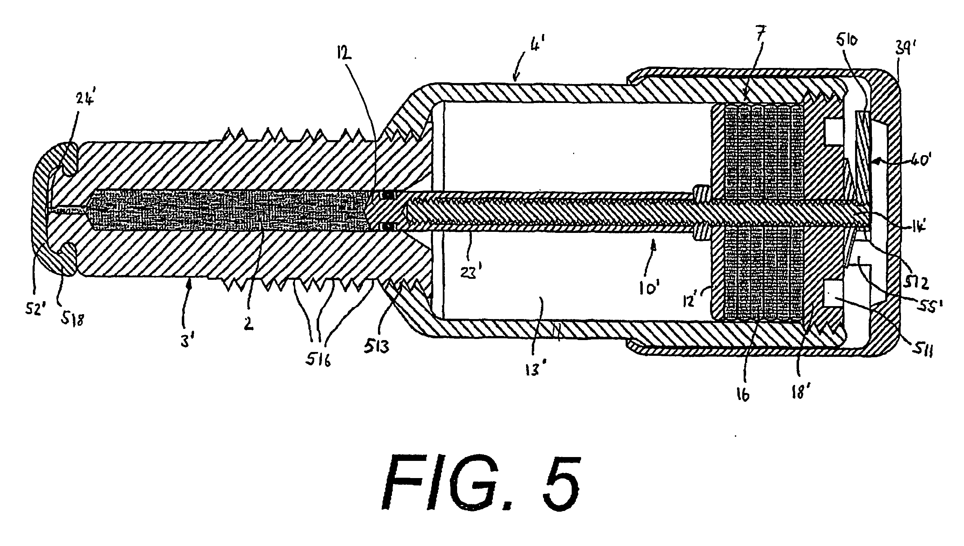

Referring to figure 5, a disposable single-use needle-free injection device outside the scope of the present invention is shown comprising a propulsion system 10', an actuator system 40', and a capsule portion 3' for the transdermal administration of a liquid 2 contained in the capsule portion.

The propulsion system comprises a housing 4', a source of potential energy 7 and a mobile force transmitting member 10'. The mobile force transmitting member comprises a propulsion system piston portion 23', a mobile force wall portion 12', and a trigger engagement portion14'. The potential energy source comprises a solid spring member 16 mounted between the mobile force wall portion 12' and a housing wall portion 18'. The potential energy used for injection is provided by the spring member which is elastically compressed between the wall portions 12', 18'. The spring members that may be used in the disposable injector are essentially the same as those described above for the re-usable device.

In this example, the piston portion 23' and trigger engagement portion 14' form substantially a rod extending along the central axis of the injection device, the mobile force wall portion being attached to the rod, or integrally formed therewith. The rod is preferably made of a steel or composite or other material with a tensile resistance sufficient to withstand the peak force generated by the compressed spring member, which may typically be in the range of 1000 to 2000 newtons. As the peak static compression force is supported by the central rod, the housing 4' and capsule portion 3' may be made of a plastic material, which advantageously maintains the weight and cost of the device low.

The mobile force wall portion is in this embodiment in the form of a plate positioned in a cavity 13' of the housing 4'. The trigger engagement portion 14' protrudes beyond a rear face of the housing pressure wall portion 18' and is fixed to a retaining element 39' of the actuator system 40'. The retaining element 39' comprises a lever portion 510 extending from a flange portion 512 attached severably to the trigger engagement portion 14' of the mobile force transmitting member 10'. In this particular example, the flange portion 512 comprises a threaded through-hole roughly about two to three thread pitches deep that engages a complementary thread of the trigger engagement portion. On applying a force on the lever portion in the axial direction that generates a moment on the flange portion, the threads of the retaining element and the trigger engagement portion shear thereby releasing the mobile pressure transmitting member 10'. The shear force on rupture of the threads will result from a combination of the force due to the compressed spring member 16, and the additional moment on pivoting the lever portion. The force required to engage the lever portion and actuate the device can thus be made quite low. Other severable means of attaching the retaining element to the trigger engagement portion may be provided, such as a rivet type attachement therebetween, a weld, or a clamp type attachment. To trigger the injection device, an actuator 39' mounted over the rear end of the device may be provided. The actuator in this example is slidable in the axial direction to press the free end of the lever portion 510. The actuator can only be pressed once it has been rotated from a safety position, where a pin 55' or other key element in abutment with the housing 4' blocks forward movement thereof, to an armed position where the pin 55' is no longer obstructed by the housing or can pass into a passage 511 therefor.

The housing 4' is connected to the capsule portion 3' by a threaded interface such that by rotating the housing with respect to the capsule portion, the dosage of liquid to be injected 2 can be reduced. For example, from a marker 516 to the successive marker there is a 0.1ml volume reduction, the initial volume being 0.5ml.

The disposable injector is further provided with a removable cap 518 to keep the application end 52' clean and sterile, and to provide additional security against inadvertent actuation of the device prior to use.

## Claims

1. A needle-free transdermal injection device with a propulsion system comprising a source of potential energy (16) and a mobile force transmitting member (10) for applying sufficient pressure on a liquid (2) to be injected for propulsing the liquid (2) to be injected through a nozzle orifice (24) at a velocity sufficient for transdermal injection, **characterized in that** the source of potential energy (16) primarily comprises a solid elastic material essentially in the form of a block elastically compressed between a wall portion (12) of the mobile force transmitting member (10) and an opposed wall portion of a housing or supporting structure of the injector (18), the solid elastic material exhibiting a non-linear characteristic of force F as a function of displacement x, whereby the ratio of the force F as a function of the displacement x increases with the compressing displacement x whereas the mobile force transmitting member (10) comprises a propulsion system piston portion (23), the wall portion (12) of the mobile force transmitting member (10) and a force generating engagement portion in the form of a screw thread portion (14) coupled to a force generating mechanism (8), the force generating mechanism comprising a first grip portion (25), a second grip portion (26) and a planetary gear mechanism (28) for driving a threaded drive tube (29) engaging the screw threaded portion (14) of the mobile force transmitting member (10) to generate the potential energy used for injection by compressing the solid elastic material between the wall portions (12, 18) by axially displacing the mobile force transmitting member (10).

2. The needle-free transdermal injection device according to the preceding claim wherein the second grip portion (26) is comprising a foldable lever portion (27).

3. The needle-free transdermal injection device according to claim 2 or 3 wherein the solid elastic material is principally reticulated polymers.

4. The needle-free transdermal injection device according to claim 3 wherein the reticulated polymer is a polyurethane.

5. The needle-free transdermal injection device according to any preceding claim wherein the solid elastic material is a cellular or alveolar construction.

6. The needle-free transdermal injection device according to any preceding claim wherein the solid elastic material is formed as a single integral block.

7. The needle-free transdermal injection device according to any of claims 1-5 wherein the solid elastic material comprises an assembly of pieces of the solid elastic material

8. The needle-free transdermal injection device according to any preceding claim wherein the potential energy source comprises a plurality of different said solid elastic materials assembled or formed together to form the spring member.

9. The needle-free transdermal injection device according to any preceding claim wherein the injector comprises at least one rod extending through the block and acting as a guide to therfor.

10. The needle-free transdermal injection device according to claim 9 wherein the block is essentially cylindrical and the rod extends through the centre of the cylinder.

## Patentansprüche

1. Nadelfreie transdermale Injektionsvorrichtung mit einem Vortriebssystem, das eine Quelle (16) potentieller Energie und ein bewegliches Kraftübertragungsorgan (10) umfasst, um auf eine zu injizierende Flüssigkeit (2) einen ausreichenden Druck auszuüben, um die zu injizierende Flüssigkeit (2) durch eine Düsenöffnung (24) mit einer für die transdermale Injektion ausreichenden Geschwindigkeit vorzutreiben, **dadurch gekennzeichnet, dass** die Quelle (16) potentieller Energie hauptsächlich ein elastisches Festkörpermaterial umfasst, das im Wesentlichen die Form eines Blocks hat, der zwischen einem Wandabschnitt (12) des beweglichen Kraftübertragungsorgans (10) und einem gegenüberliegenden Wandabschnitt eines Gehäuses oder einer Unterstützungsstruktur des Injektors (18) elastisch komprimiert ist, wobei das elastische Festkörpermaterial eine nichtlineare Kraftcharakteristik F als Funktion der Verlagerung x hat, wobei das Verhältnis der Kraft F als Funktion der Verlagerung x mit der Kompressionsverlagerung x ansteigt, wobei das bewegliche Kraftübertragungsorgan (10) einen Vortriebsystem-Kolbenabschnitt (23), den Wandabschnitt (12) des beweglichen Kraftübertragungsorgans (10) und einen Krafterzeugungs-Eingriffabschnitt in Form eines Schraubgewindeabschnitts (14), der mit einem Krafterzeugungsmechanismus (8) gekoppelt ist, umfasst, und der Krafterzeugungsmechanismus einen ersten Greifabschnitt (25), einen zweiten Greifabschnitt (26) und einen Planetengetriebemechanismus (28) umfasst, um ein Gewindeantriebsrohr (29), das mit dem Schraubgewindeabschnitt (14) des beweglichen Kraftübertragungsorgans (10) in Eingriff ist, anzutreiben, um die potentielle Energie zu erzeugen, die für die Injektion verwendet wird, indem das elastische Festkörpermaterial zwischen den Wandabschnitten (12, 18) durch axiale Verlagerung des beweglichen Kraftübertragungsorgans (10) komprimiert wird.

2. Nadelfreie transdermale Injektionsvorrichtung nach dem vorhergehenden Anspruch, wobei der zweite Griffabschnitt (26) einen klappbaren Hebelabschnitt (27) umfasst.

3. Nadelfreie transdermale Injektionsvorrichtung nach Anspruch 2 oder 3, wobei das elastische Festkörpermaterial hauptsächlich netzartige Polymere enthält.

4. Nadelfreie transdermale Injektionsvorrichtung nach Anspruch 3, wobei das netzartige Polymer Polyurethan ist.

5. Nadelfreie transdermale Injektionsvorrichtung nach einem der vorhergehenden Ansprüche, wobei das elastische Festkörpermaterial eine zellulare oder alveoläre Konstruktion ist.

6. Nadelfreie transdermale Injektionsvorrichtung nach einem der vorhergehenden Ansprüche, wobei das elastische Festkörpermaterial als ein einziger integraler Block ausgebildet ist.

7. Nadelfreie transdermale Injektionsvorrichtung nach einem der Ansprüche 1-5, wobei das elastische Festkörpermaterial eine Baugruppe von Teilen des elastischen Festkörpermaterials umfasst.

8. Nadelfreie transdermale Injektionsvorrichtung nach einem der vorhergehenden Ansprüche, wobei die Quelle potentieller Energie mehrere verschiedene elastische Festkörpermaterialien umfasst, die zusammengefügt oder angeordnet sind, um das Federorgan zu bilden.

9. Nadelfreie transdermale Injektionsvorrichtung nach einem der vorhergehenden Anspruch, wobei der Injektor wenigstens einen Stab aufweist, der sich durch den Block erstreckt und als eine Führung hierfür wirkt.

10. Nadelfreie transdermale Injektionsvorrichtung nach Anspruch 9, wobei der Block im Wesentlichen zylindrisch ist und der Stab sich durch die Mitte des Zylinders erstreckt.

## Revendications

1. Dispositif d'injection transdermique sans aiguille avec un système de propulsion comprenant une source d'énergie potentielle (16) et un élément de transmission de force mobile (10) pour appliquer une pression suffisante sur un liquide (2) à injecter pour la propulsion du liquide (2) à injecter à travers un orifice de tuyère (24) à une vitesse suffisante pour l'injection transdermique, **caractérisé en ce que** la source d'énergie potentielle (16) comprend principalement un matériau élastique solide sensiblement sous la forme d'un bloc comprimé élastiquement entre une portion de paroi (12) de l'élément de transmission de force mobile (10) et une portion de paroi opposée d'un logement ou d'une structure de support de l'injecteur (18), le matériau élastique solide présentant une caractéristique non linéaire de force F en fonction du déplacement x, moyennant quoi le rapport de la force F en fonction du déplacement x augmente avec le déplacement de compression x, tandis que l'élément de transmission de force mobile (10) comprend une portion de piston de système de propulsion (23), la portion de paroi (12) de l'élément de transmission de force mobile (10) et une portion d'engagement générant la force sous forme d'une portion de tête de vis (14) couplée à un mécanisme de génération de force (8), le mécanisme de génération de force comprenant une première portion de préhension (25), une seconde portion de préhension (26) et un mécanisme à engrenage planétaire (28) pour entraîner un tube de commande fileté (29) venant en prise avec la portion filetée de vis (14) de l'élément de transmission de force mobile (10), pour générer l'énergie potentielle utilisée pour l'injection en comprimant le matériau élastique solide entre les portions de paroi (12, 18) par déplacement axial de l'élément de transmission de force mobile (10).

2. Dispositif d'injection transdermique sans aiguille selon la revendication précédente, dans lequel la seconde portion de préhension (26) comprend une portion de levier pliable (27).

3. Dispositif d'injection transdermique sans aiguille selon la revendication 2 ou 3, dans lequel le matériau élastique solide est principalement constitué de polymères réticulés.

4. Dispositif d'injection transdermique sans aiguille selon la revendication 3, dans lequel le polymère réticulé est un polyuréthane.

5. Dispositif d'injection transdermique sans aiguille selon l'une quelconque des revendications précédentes, dans lequel le matériau élastique solide consiste en une structure cellulaire ou alvéolaire.

6. Dispositif d'injection transdermique sans aiguille selon l'une quelconque des revendications précédentes, dans lequel le matériau élastique solide est formé d'un bloc en une seule pièce.

7. Dispositif d'injection transdermique sans aiguille selon l'une quelconque des revendications 1 à 5, dans lequel le matériau élastique solide comprend un ensemble de pièces de matériau élastique solide.

8. Dispositif d'injection transdermique sans aiguille selon l'une quelconque des revendications précédentes, dans lequel la source d'énergie potentielle comprend une pluralité de différents matériaux élastiques solides assemblés ou formés ensemble pour constituer l'élément à ressort.

9. Dispositif d'injection transdermique sans aiguille selon l'une quelconque des revendications précédentes, dans lequel l'injecteur comprend au moins une tige s'étendant à travers le bloc et agissant comme guide.

10. Dispositif d'injection transdermique sans aiguille selon la revendication 9, dans lequel le bloc est sensiblement cylindrique et la tige s'étend à travers le centre du cylindre.
